# EUROPÄISCHE PATENTSCHRIFT

(11) **EP 2 008 597 B1**
(45) Veröffentlichungstag und Bekanntmachung des Hinweises auf die Patenterteilung: **21.09.2011**
(21) Anmeldenummer: 07111032.4
(22) Anmeldetag: 26.06.2007
(51) Int. Cl.: A61B 17/15, A61B 19/00

(54) **Vorjustierung von justierbaren Knochenschneidblöcken zum Ermöglichen einer Navigation der Schnittebene bezüglich Referenzobjekten**
Preadjustment of adjustable bone cutting blocks to allow for navigation of the cutting place with regard to reference objects
Préajustement de blocs de coupe d'os ajustables destiné à permettre la navigation du plan de coupe relatif à des objets de référence

(43) Veröffentlichungstag der Anmeldung: 31.12.2008
(73) Patentinhaber: BrainLAB AG, 85622 Feldkirchen (DE)
(72) Erfinder: Götte, Hubert, Dr., 80333, München (DE); Kluzik, Jacek, Dr., 85579, Neubiberg (DE)
(74) Vertreter: Schwabe - Sandmair - Marx

(56) Entgegenhaltungen:
- EP-A- 1 690 503

## Beschreibung

Die vorliegende Erfindung betrifft einen justierbaren Schneidblock, insbesondere einen justierbaren Knochenschneidblock, welcher vor seiner Justierung vorjustiert werden kann, um bei der Einstellung der durch die Lage seiner Schneidführung festgelegte Schnittebene eine Navigation bezüglich Referenzobjekten zu ermöglichen. Weiter bezieht sich die vorliegende Offenbarung auf ein System und ein Verfahren zur Navigation der Schnittebene der Schneidführung des Schneidblocks bezüglich Referenzobjekten eines Körpers eines Patienten.

Beispielsweise bei der computerunterstützten Knie-Arthroplastie wird gewöhnlich ein Schneidblock verwendet, der eine Schneidführung aufweist, an welcher eine mitunter räumlich verstellbare z.B. drehbare Ortungsreferenz, wie ein Infrarot-Strahlung emittierender oder reflektierender Referenzstern, angebracht ist, um eine bestmögliche Sichtbarkeit der Ortungsreferenz für verschiedene Setups zu erlauben. Mittels der Ortungsreferenz und eines medizintechnischen Navigationssystems wird versucht, den Schneidblock so zu positionieren, dass die Schneidführung in der geplanten Schnittebene zu liegen kommt.

Aus der EP 1 690 503 A1 der Anmelderin ist ein Knochenschneidblock bekannt, welcher eine Schneidführung umfasst, an welcher verstellbar z.B. drehbar eine Ortungsreferenz angebracht ist, die es gestattet, die Schnittebene der Schneidführung räumlich zu bestimmen, welcher weiter eine Befestigung umfasst, die an einem Knochen befestigt werden kann, welcher eine Justierungseinrichtung zwischen Befestigung und Schneidführung umfasst, wobei mittels der Justierungseinrichtung die Schnittebene der Schneidführung gegenüber dem Knochen eingestellt werden kann, und welcher ein Registrierungselement umfasst, mit dem die räumliche Lage der Justierungseinrichtung bestimmbar ist.

Die Justierungseinrichtung weist Einstellmittel, wie Handrad-Schraubeinstellvorrichtungen, auf, mittels denen die Schneidführung um zwei nichtparallele Achsen gedreht und in der Höhe verstellt werden kann, wobei jeweils ein Einstellmittel zur Veränderung eines mechanischen Freiheitsgrades der Schneidführung dienen soll. Die Veränderung eines mechanischen Freiheitsgrades der Schneidführung führt bezüglich eines Knochen-Koordinatensystems bezüglich dessen die Schneidführung oder die Schnittebene der Schneidführung definiert ist zur Veränderung des entsprechenden Freiheitsgrades der Schnittebene. Damit ist mit einem solchen herkömmlichen Schneidblock zwar eine so genannte Navigation bezüglich eines vorbestimmten Plans ("navigation to plan") möglich, bei welcher eine Ist-Schnittebene mit Hilfe eines Navigationssystems auf die vorgegebene räumliche Lage einer Soll-Schnittebene ausgerichtet oder eingestellt wird. Eine so genannte Navigation zur Referenz oder Navigation bezüglich einer oder mehrerer Referenzen ("navigation to reference"), bei welcher die Schnittebene in einem beispielsweise durch definierte Projektionsebenen bestimmten Referenzkoordinatensystem bezüglich präoperativ bestimmten Referenzen, wie charakteristischer Ebenen oder Achsen oder Punkte eines Körpers, ausgerichtet werden soll, kann mit einem solchen Schneidblock nicht oder nur mit einer Vielzahl von Iterationen durch wiederholtes Einstellen der Einstellmittel durchgeführt werden. Der Grund hierfür liegt darin, dass Veränderungen an einem Einstellmittel der Justierungseinrichtung nicht nur eine Veränderung des gewünschten Freiheitsgrades der Ist-Schnittebene in dem Referenzkoordinatensystem relativ zu den Referenzen bewirkt, sondern zusätzlich wenigstens ein weiterer Freiheitsgrad der Ist-Schnittebene relativ zu den Referenzen verändert wird. Sollen beispielsweise bestimmte Abstände der Ist-Schnittebene des Schneidblocks relativ zu Ebenen oder Achsen oder Punkten des Körpers eingestellt oder eingehalten werden und wurde ein erster Abstand durch wiederholtes Justieren einer Vielzahl der Einstellmittel eingestellt, wird durch den Versuch durch Veränderung des jeweiligen Einstellmittels einen zweiten Abstand der Schnittebene oder der Schneidführung relativ zu einer weiteren Achse oder Ebene oder einem weiteren Punkt einzustellen, der erste Abstand mitverstellt. Damit ist die exakte Einstellung der Ist-Schnittebene bezüglich der Referenzen in dem Referenz-Koordinatensystem häufig mit einer großen Anzahl an Iterationen verbunden.

Es ist eine Aufgabe der vorliegenden Erfindung die Nachteile aus dem Stand der Technik zu überwinden. Es ist weiter eine Aufgabe der vorliegenden Erfindung einen Schneidblock zur Navigation der Schnittebene der Schneidführung des Schneidblocks bezüglich Referenzobjekten eines Körpers eines Patienten bereitzustellen, welche eine schnelle und genaue Navigation der Schnittebene des Schneidblocks bezüglich Referenzen oder Referenzobjekten eines Körpers ermöglichen.

Diese Aufgabe wird durch den Gegenstand des unabhängigen Anspruchs gelöst. Vorteilhafte Ausführungsformen ergeben sich aus den abhängigen Ansprüchen.

Eine Navigation bezüglich Referenzen oder Referenzobjekten eines Körpers bedeutet, dass die Schnittebene oder Ist-Schnittebene einer Schneidführung eines Scheidblocks in einem Referenz-Koordinatensystem bezüglich charakteristischer oder prä- oder intraoperativ bestimmter oder ausgewählter Referenzobjekte eines Körpers, wie charakteristischer Ebenen, z.B. des Tibiaplateaus, der Sagittal-, Frontal- oder Transversalebene, Achsen, z.B. der Femur- oder Tibiaachse, oder Punkte, z.B. der Ansätze der Kreuzbänder, eines Körpers eingestellt oder navigiert wird oder werden kann, so dass die Ist-Schnittebene beispielsweise von einem Operateur angegebene oder vorgegebene Abstände von den Ebenen, Achsen oder Punkten des Körpers einnimmt oder einhält. Der Vorteil dieser Navigationsweise liegt darin, dass man von den Erfahrungswerten des Operateurs profitieren kann, welcher auf Grund seiner Erfahrung weiß welche Abstände die Schneidführung oder die Schnittebene der Schneidführung zu den Referenzen haben oder einhalten muss, damit die Schneidführung optimal platziert ist. Eine Vielzahl von Operateuren bevorzugt eine Navigation bezüglich Referenzobjekten, da hierbei die tatsächlich gewünschten Abstände von den Referenzobjekten direkt eingestellt werden können. Zudem entfällt bei der Navigation bezüglich Referenzen die aufwendige Planungsprozedur zur Erstellung eines Plans, bezüglich dessen navigiert werden soll.

Der erfindungsgemäße Schneidblock oder Schnittblock, insbesondere Knochenschneidblock, umfasst eine Schneidführung, an welcher verstellbar, insbesondere drehbar, eine Ortungsreferenz, z.B. ein Infrarot-Strahlung emittierender oder reflektierender Referenzstern, angebracht ist, mittels welcher die Ist-Schnittebene der Schneidführung oder die Position und/oder Orientierung oder Lage der Schneidführung und damit der Ist-Schnittebene räumlich bestimmt werden kann. Weiter umfasst der Schneidblock eine Befestigung, die an einem Knochen befestigt werden kann, eine vorzugsweise zwischen Befestigung und Schneidführung angeordnete oder ausgebildete Justierungseinrichtung, wobei mittels der Justierungseinrichtung die Schneidführung und damit die Ist-Schnittebene der Schneidführung gegenüber dem Knochen mit zwei rotatorischen Freiheitsgraden und einem translatorischen Freiheitsgrad eingestellt werden kann und eine Ortungsreferenz, insbesondere ein temporär oder vorübergehend an der Justierungseinrichtung angeordneter Referenzstern, mittels der die räumlichen Lage der Justierungseinrichtung, insbesondere relativ zu dem Knochen-Koordinatensystem oder zu dem Knochen, bestimmt werden kann. Dabei sind die Referenzen oder Referenzobjekte in einem Navigationssystem bezüglich eines Referenz- oder Referenznavigations- oder Projektions- oder Bild-Koordinatensystems definiert, so dass die Lage oder die Position und/oder Orientierung der Referenzen oder Referenzobjekte bezüglich des Referenz-Koordinatensystem dem Navigationssystem oder einer mit dem Navigationssystem drahtlos oder drahtgebunden verbundenen oder in das Navigationssystem integrierten Recheneinheit bekannt ist. Insbesondere wird das Referenz-Koordinatensystem durch zwei senkrecht aufeinander stehende Bildebenen, wie die Frontal- oder Sagittalebene eines Körperteils oder Körpers des Patienten, gebildet oder aufgespannt. Die mittels des Navigationssystems über oder mit Hilfe der Ortungsreferenz ermittelte Ist-Schnittebene oder Position und/oder Orientierung der Ist-Schnittebene oder Schneidführung ist bezüglich des Knochen-Koordinatensystems definiert oder wird durch das Knochen-Koordinatensystem definiert oder beschrieben, so dass dem Navigationssystem die Lage der Ist-Schnittebene oder die Position und/oder Orientierung der Ist-Schnittebene oder der Schneidführung bezüglich des Knochen-Koordinatensystem im Raum bekannt ist. Werden die Referenzobjekte oder die Positionen und/oder Orientierungen der Referenzobjekte in dem Knochen-Koordinatensystem oder bezüglich des Knochen-Koordinatensystems ausgewählt oder gewählt, so ist dem Navigationssystem die Lage der Referenzen oder der Referenzobjekte in dem Knochen-Koordinatensystem oder bezüglich des Knochen-Koordinatensystems bekannt. Sind oder werden die Referenzobjekte bezüglich des Referenz-Koordinatensystems definiert oder wird das Referenz-Koordinatensystem durch die Referenzobjekte gebildet, so kann von dem Navigationssystem eine Transformationsvorschrift zwischen dem Knochen- und dem Referenz-Koordinatensystem ermittelt werden, so dass das Knochen-Koordinatensystem in das Referenz-Koordinatensystem überführbar ist und umgekehrt das Referenz-Koordinatensystem in das Knochen-Koordinatensystem überführbar ist. Somit sind die Lage der Ist-Schnittebene und der Referenzobjekte in dem Referenz-Koordinatensystem ermittelbar und darstellbar und die Lage der Ist-Schnittebene und der Referenzobjekte in dem Knochen-Koordinatensystem ermittelbar und darstellbar.

Da die Drehachsen des Referenz-Koordinatensystems und der Mechanik des Schneidblocks oder der Justierungseinrichtung in der Regel verschieden sind, führt eine Veränderung eines an der Justierungseinrichtung angeordneten Einstellmittels zur Einstellung einer Richtung oder eines mechanischen Freiheitsgrades der Schneidführung bezüglich des Referenz-Koordinatensystems zumeist auch zu Veränderungen der Ist-Schnittebene in mehr als einem Freiheitsgrad oder in weiteren als dem mit dem Einstellmittel zu verändern beabsichtigten Freiheitsgrad, so dass nicht nur der Freiheitsgrad verändert wird, welcher z.B. zum Einstellen eines gewünschten Abstands bezüglich einer Referenz verstellt werden sollte. Dies führt zu einer erheblichen Erschwerung der Ausrichtung der Ist-Schnittebene bezüglich der Referenzen oder Referenzobjekte in dem Referenz-Koordinatensystem und erfordert zumeist ein Nachjustieren in vielen Schritten, so dass eine effektive Navigation bezüglich Referenzen nicht oder nur mit einem für praktische Anwendungen zu hohem Zeitaufwand möglich ist.

Der erfindungsgemäße Schneidblock umfasst weiter eine Vorjustierungseinrichtung, mittels welcher das Basis-Koordinatensystem bezüglich des Referenz-Koordinatensystems mit zwei rotatorischen Freiheitsgraden so ausgerichtet werden kann, dass eine erste Achse des Basis-Koordinatensystems parallel zu einer durch zwei Achsen des Referenz-Koordinatensystems aufgespannten Ebene verläut und eine zweite Achse des Basis-Koordinatensystems parallel zu der dritten Achsen des Referenz-Koordinatensystems verläut. Die Vorjustierungseinrichtung umfasst hierfür vorzugsweise Vorjustierungs-Einstellmittel, insbesondere jeweils ein Einstellmittel für jeden Freiheitsgrad, speziell zwei Handrad-Einstellschrauben oder arretierbare Schrauben, mittels welchen das Basis-Koordinatensystem bezüglich des Referenz-Koordinatensystems ausgerichtet werden kann. Vorzugsweise weist die Vorjustierungseinrichtung Gelenke, wie Kugelgelenke oder insbesondere zumindest zwei Scharniergelenke, auf, um welche die Drehung oder Ausrichtung erfolgen kann. Während des Vorjustierungsvorganges ist bevorzugt ein Referenzstern an dem Basis-Koordinatensystem des Schneidblocks oder an dem Schneidblock angeordnet, so dass die Ausrichtung des Basis-Koordinatensystems mittels eines Navigationssystems festgestellt werden kann.

Eine Vorjustierung mittels der Vorjustierungseinrichtung wird so lange durchgeführt bis das Basis-Koordinatensystem zumindest nahezu auf das oder relativ zu dem Referenz-Koordinatensystem oder bezüglich des Referenz-Koordinatensystems ausgerichtet ist oder wurde. Unter der zumindest nahezu vollständigen Ausrichtung ist nicht zwangsläufig zu verstehen, dass das Basis-Koordinatensystem nach der Ausrichtung exakt auf dem Referenz-Koordinatensystem liegen soll oder alle Achsen des Basis-Koordinatensystems parallel zu den Achsen des Referenz-Koordinatensystem liegen müssen. Vorzugsweise ist die Vorjustierung abgeschlossen oder erfolgreich, wenn eine erste Achse des Basis-Koordinatensystems parallel zu einer durch zwei Achsen des Referenz-Koordinatensystems aufgespannten Ebene verläuft und eine zweite Achse des Basis-Koordinatensystems parallel zu der dritten Achse des Referenz-Koordinatensystems verläuft. Beispielsweise können nach der so erfolgten Ausrichtung zwei Achsen des Basis-Koordinatensystems nicht parallel zu den entsprechenden Achsen des Referenz-Koordinatensystems verlaufen. Auch dann ist das Basis-Koordinatensystem zumindest nahezu auf das oder relativ zu dem Referenz-Koordinatensystem ausgerichtet. Selbstverständlich können aber auch das Referenz- und das Basis-Koordinatensystem exakt aufeinander oder relativ zueinander ausgerichtet sein oder werden.

Nach der erfolgten Ausrichtung des Basis-Koordinatensystems bezüglich des Referenz-Koordinatensystems werden die Vorteile der vorliegenden Offenbarung deutlich.

Ist das Basis-Koordinatensystem zumindest nahezu auf das Referenz-Koordinatensystem ausgerichtet, ist eine einfache und schnelle Navigation der Ist-Schnittebene bezüglich des Referenz-Koordinatensystems oder bezüglich der Referenzobjekte des Körpers in dem Referenz-Koordinatensystem möglich, so dass eine Navigation bezüglich der Referenzen oder Referenzobjekte in wenigen Schritten oder Iterationen durchgeführt werden kann. Die nach dem Ausrichten durch die Einstellung der Einstellmittel der Justierungseinrichtung durchgeführten Veränderungen oder Bewegungen der Schneidführung können somit zumindest teilweise als voneinander entkoppelte Veränderungen der Freiheitsgrade der Ist-Schnittebene bezüglich der Referenzen in dem Referenz-Koordinatensystem oder bezüglich des Referenz-Koordinatensystems in dem Navigationssystem ermittelt und dargestellt werden, so dass zumindest eine an der Justiereinrichtung durchgeführte Veränderung eines mechanischen Freiheitsgrads der Schneidführung nur eine Veränderung des gleichen Freiheitsgrades der Ist-Schnittebene in dem Referenz-Koordinatensystem oder relativ zu den Referenzobjekten in dem Referenz-Koordinatensystem bewirkt. Bei der Einstellung eines ersten mechanischen Freiheitsgrades der Schneidführung werden sowohl der gewünschte oder korrespondierende Freiheitsgrad der Ist-Schnittebene in dem Referenz-Koordinatensystem entsprechend der Einstellung des mechanischen Freiheitsgrades der Schneidführung verändert, als auch ein zweiter Freiheitsgrad der Ist-Schnittebene in dem Referenz-Koordinatensystem mitverändert. Ist jedoch der erste Freiheitsgrad der Ist-Schnittebene zumindest nahezu, vorzugsweise genau, entsprechend der Veränderung des mechanischen Freiheitsgrades der Schneidführung eingestellt, so bewirkt eine Veränderung eines zweiten mechanischen Freiheitsgrades der Schneidführung zumindest nahezu, vorzugsweise genau, nur eine Veränderung des zweiten korrespondierenden Freiheitsgrades der Ist-Schnittebene in dem Referenz-Koordinatensystem, ohne dass der erste Freiheitsgrad der Ist-Schnittebene mitverändert wird. Die Einstellmittel der Justierungseinrichtung können dann beispielsweise in wenigen Schritten oder ohne Nachjustieren so eingestellt werden, dass die Ist-Schnittebene in dem Referenz-Koordinatensystem bezüglich bestimmter Ebenen, oder Achsen oder Punkte des Körpers vorgegebene oder von dem Operateur angegebene Werte oder Abstände hat oder einhält oder dass die Ist-Schnittebene oder eine Projektion der Ist-Schnittebene mit charakteristischen Achsen und/oder Ebenen eines Körpers Winkel ausbildet, wie den Varus-Valguswinkel oder den Flexions-Extensionswinkel, welche bestimmte vorgegebene Winkelwerte annehmen oder einhalten. Dies ermöglicht eine einfache und schnelle Navigation der Schnittebene der Schneidführung relativ zu Referenzen in wenigen Schritten und ohne eine Vielzahl von Iterationen.

Auf Grund der Ausrichtung des Basis-Koordinatensystems bezüglich des Referenz-Koordinatensystems kann nach Einstellen eines ersten Freiheitsgrades der Ist-Schnittebene (10) der Schneidführung (1) durch Veränderung eines ersten Einstellmittels der Justierungseinrichtung (20), erreicht werden, dass durch Veränderung eines weitem Einstellmittels der Justierungseinrichtung in dem Referenz-Koordinatensystem stets nur eine Veränderung der Ist-Schnittebene in einem Freiheitsgrad erfolgt. Dadurch wird verhindert, dass die Einstellung eines Einstellmittels der Justierungseinrichtung die Einstellung eines weiteren Einstellmittels in dem Referenz-Koordinatensystem beeinflusst oder zumindest teilweise rückgängig macht. Beispielsweise kann ein erster Freiheitsgrad der Ist-Schnittebene, wie eine erste Drehung oder eine erste Rotation, mittels eines ersten Einstellmittels oder einer ersten Handrad-Einstellschraube beispielsweise verändert oder eingestellt werden, bis mittels des Navigationssystems oder an dem Einstellmittel angezeigt wird, dass der gewünschte Abstand oder Winkelwert relativ zu einer Referenz erreicht ist. Bei dieser Einstellung ist zumindest ein Freiheitsgrad der Ist-Schnittebene noch nicht entkoppelt von dem ersten Freiheitsgrad der Ist-Schnittebene, welcher durch das erste Einstellmittel eingestellt wird. Ist der erste Freiheitsgrad eingestellt, kann mittels eines zweiten Einstellmittels ein zweiter Freiheitsgrad der Ist-Schnittebene, wie ein weiterer Rotationsfreiheitsgrad, von einem Benutzer wie gewünscht verändert werden, ohne dass dies zu einer Veränderung oder Beeinflussung des ersten Freiheitsgrades führt, so dass zielgerichtet ein zweiter Abstands- oder Winkelwert relativ zu einer Referenz eingestellt werden kann. Auch die Veränderung eines dritten Freiheitsgrades, beispielsweise eines translatorischen Freiheitsgrades, führt nicht zu einer Veränderung des ersten und zweiten Freiheitsgrades der Schnittebene oder des eingestellten ersten und zweiten Abstands oder Winkelwertes in dem Referenzkoordinatensystem. Damit kann bei der Verwendung von drei Einstellmitteln vorzugsweise in drei Einstellschritten die Schneidführung oder die Ist-Schnittebene der Schneidführung bezüglich der Referenzen eingestellt werden.

Vorzugsweise ist die Vorjustierungseinrichtung zwischen der Befestigung des Schneidblocks und der Justierungseinrichtung ausgebildet und umfasst bevorzugt Gelenke, insbesondere Kugelgelenke oder zumindest zwei Scharniere, um welche die Bewegung oder Drehung des Basis-Koordinatensystems erfolgen kann.

Die Offenbarung betrifft weiter ein System zur Navigation der Schneidführung des erfindungsgemäßen Schneidblocks bezüglich Referenzen eines Körpers eines Patienten. Mit dem System kann der erfindungsgemäße Schneidblock so vorbereitet oder eingestellt werden, dass er zur schnellen und einfachen Navigation bezüglich Referenzobjekten des Körpers des Patienten geeignet ist oder verwendet werden kann, so dass nach abgeschlossener Vorbereitung des Schneidblocks eine Navigation der Schneidführung oder der Ist-Schnittebene des Schneidblocks bezüglich der Referenzobjekte oder Referenzen, wie Ebenen, Achsen, z.B. der Femur- oder Tibiaachse, oder Punkte eines Körpers erfolgen kann. Das System umfasst ein Navigationssystem mit einer mit dem Navigationssystem drahtlos oder drahtgebunden verbundenen oder in das Navigationssystem integrierten Recheneinheit, insbesondere einem Computer oder Prozessor. Das Navigationssystem kann, vorzugsweise mittels IR-Kameras, an der Schneidführung und vorübergehend oder temporär an der Justierungseinrichtung des Schneidblocks angeordnete Ortungsreferenzen, wie IR-Strahlung reflektierende oder emittierende Referenzsterne, oder Registrierungselemente detektieren. Aus den erfassten Ortungsreferenzen kann die Recheneinheit die Lage oder die Position und/oder Orientierung der Ist-Schnittebene der Schneidführung, der Justierungseinrichtung und der Vorjustierungseinrichtung, insbesondere bezüglich des Knochens, ermitteln, so dass dem Navigationssystem die absolute Lage der Ist-Schnittebene, der Justierungseinrichtung und der Vorjustierungseinrichtung und die relative Lage der Ist-Schnittebene, der Justierungseinrichtung und der Vorjustierungseinrichtung zueinander bekannt ist.

Dem Navigationssystem ist das Knochen-Koordinatensystem bekannt oder das Knochen-Koordinatensystem kann von dem Navigationssystem ermittelt werden, wobei bezüglich des Knochen-Koordinatensystems die Position und/oder Orientierung der Schneidführung des Schneidblocks oder der Ist-Schnittebene der Schneidführung definiert ist, so dass die Position und/oder Orientierung der Ist-Schnittebene auch in dem Referenz-Koordinatensystem ermittelbar sind. Insbesondere kann die Lage der Schneidführung oder Ist-Schnittebene durch das Knochen-Koordinatensystem bestimmt sein.

Auch kann das Basis-Koordinatensystem frei wählbar sein oder kann sich bevorzugt zwischen der Vorjustierungs- und Justierungseinrichtung, insbesondere an deren Verbindungsstelle befinden. Weiter ist dem Navigationssystem das Referenz-Koordinatensystem bekannt oder kann von diesem ermittelt werden, wobei bezüglich des Referenz-Koordinatensystems die Position und/oder Orientierung von Referenzobjekten des Körpers, wie Referenzpunkten, Referenzachsen oder -geraden oder Referenzebenen, definiert ist. Das Referenz-Koordinatensystem befindet sich bevorzugt auf oder in dem Körper des Patienten, insbesondere an dem Knochen, an welchem der Schneidblock angeordnet ist und wird bevorzugt durch Projektionsebenen, wie die Frontal- oder Sagittalebene bestimmt oder gebildet.

Mit der Vorjustierungseinrichtung kann nun, insbesondere durch Verstellung der vorzugsweise drei Einstellmittel der Vorjustierungseinrichtung das Basis-Koordinatensystem relativ zu dem Referenz-Koordinatensystem zumindest nahezu ausgerichtet werden. Sind das Basis-Koordinatensystem und das Referenz-Koordinatensystem nicht bezüglich einander zumindest nahezu ausgerichtet, so bewirkt ein Verstellen eines Einstellmittels der Justierungseinrichtung, welches bevorzugt zu einer Veränderung eines mechanischen Freiheitsgrades der Schneidführung insbesondere relativ zu dem Basis-Koordinatensystem vorgesehen ist, relativ zu dem Referenz-Koordinatensystem nicht nur eine Veränderung des gewünschten Freiheitsgrades der Schneidführung oder der Ist-Schnittebene des Schneidblocks sondern auch eine Veränderung wenigstens eines zusätzlichen Freiheitsgrades der Schnittebene. Dadurch können vorgegebene Abstände der Ist-Schnittebene von bestimmten Referenzen des Körpers nur durch eine Vielzahl von Iterationen eingestellt werden oder nur ungenau oder gar nicht eingestellt werden.

Ist das Basis-Koordinatensystem durch Einstellen oder Verändern der Einstellmittel der Vorjustierungseinrichtung zumindest nahezu bezüglich des Referenz-Koordinatensystems ausgerichtet bzw. ausgerichtet worden, werden Vorteile deutlich. Nach dem Ausrichten der Koordinatensysteme relativ zueinander bewirkt eine Verstellung eines zur Veränderung eines mechanischen Freiheitsgrades der Schneidführung dienenden Einstellmittels auch in dem Referenzkoordinatensystem zumindest teilweise nur eine Veränderung der Ist-Schnittebene in nur einem Freiheitsgrad relativ zu den Referenzen, so dass eine einfache, gezielte und schnelle Einstellung der vorgegebenen Abstände der Ist-Schnittebene relativ zu den gewählten oder vorbestimmten Referenzen des Körpers gewährleistet wird. Die entkoppelte Veränderung oder Verstellung der Freiheitsgrade ist dann möglich, wenn das Basis-Koordinatensystem zumindest nahezu bezüglich des Referenz-Koordinatensystems ausgerichtet ist und ein erster mechanischer Freiheitsgrad der Schneidführung bereits wie gewünscht eingestellt wurde. Bei dem Einstellen des ersten mechanischen Freiheitsgrades der Schneidführung wird ein erster zu dem ersten mechanischen Freiheitsgrad der Schneidführung korrespondierender Freiheitsgrad der Ist-Schnittebene und zudem zumindest ein zweiter Freiheitsgrad verändert. Ist der erste mechanische Freiheitsgrad wie gewünscht eingestellt und wird ein zweiter mechanischer Freiheitsgrad verändert, bewirkt dessen Veränderung nur eine Veränderung des korrespondierenden zweiten Freiheitsgrades der Ist-Schnittebene in dem Referenz-Koordinatensystem entkoppelt von dem ersten Freiheitsgrad und den weiteren Freiheitsgraden der Ist-Schnittebene. Alle weiteren Freiheitsgrade können nun entkoppelt voneinander eingestellt oder verstellt werden. Zur wunschgemäßen Einstellung der Freiheitsgrade bestimmt die Recheneinheit die Lage oder die Position und/oder Orientierung der Ist-Schnittebene der Schneidführung relativ zu den der Recheneinheit bekannten oder von der Recheneinheit ermittelten Positionen und/oder Orientierungen der Referenzobjekte. Die relative Lage oder der relative Abstand der Ist-Schnittebene von den Referenzobjekten wird von der Recheneinheit bestimmt und vorzugsweise auf einer Anzeigevorrichtung, wie einem Monitor oder Bildschirm, angezeigt oder ausgegeben. Beispielsweise können Zahlenwerte ausgegeben werden, welche die Abstände der Ist-Schnittebene von den jeweiligen Referenzen symbolisieren, anhand welcher der Operateur erkennen kann, ob die von ihm gewünschten Abstände der Ist-Schnittebene von den Referenzobjekten erreicht sind oder wie weit die tatsächlichen Abstände von den gewünschten Abständen abweichen. Auch kann die Position und/oder Orientierung der Ist-Schnittebene relativ zu den Referenzobjekten zusätzlich zu den Zahlenwerten grafisch oder schematisch auf der Anzeigevorrichtung dargestellt werden. Durch Verstellen der Einstellmittel der Justierungseinrichtung kann der Operateur nun gezielt vorzugsweise jeden weiteren Freiheitsgrad der Ist-Schnittebene relativ zu dem Referenzkoordinatensystem unabhängig oder entkoppelt von anderen Freiheitsgraden verstellen bis die gewünschten Abstände der Ist-Schnittebene von den Referenzobjekten eingestellt sind oder vorliegen. Auch kann nach Einstellen des ersten Freiheitsgrades bevorzugt mit jedem Einstellmittel der Justierungseinrichtung ein einziger Freiheitsgrad der Ist-Schnittebene entkoppelt von den anderen Freiheitsgraden relativ zu oder in dem Referenzkoordinatensystem oder relativ zu den Referenzen verstellt werden.

Die Offenbarung betrifft weiter ein Verfahren zur Navigation einer Schneidführung des erfindungsgemäßen Schneidblocks bezüglich Referenzobjekten eines Körpers eines Patienten. Dabei werden mittels eines Navigationssystem, insbesondere mittels IR-Kameras des Navigationssystems, an der Schneidführung und temporär an der Justierungseinrichtung angeordnete Ortungsreferenzen oder Registrierungselemente erfasst und die Lage oder die Position und/oder Orientierung der Ist-Schnittebene der Schneidführung und der Justierungseinrichtung wird mittels einer mit dem Navigationssystem verbundenen oder in das Navigationssystem integrierten Recheneinheit bezüglich eines Knochen-Koordinatensystems ermittelt. Mittels an der Vorjustierungseinrichtung angeordneten Einstellmitteln wird ein Basis-Koordinatensystem, bezüglich dessen die Lage der Justierungseinrichtung definiert ist, verstellt oder verändert, insbesondere translatorisch oder rotatorisch bewegt. Das Basis-Koordinatensystem wird so lange oder so weit bewegt, bis es bezüglich eines Referenz-Koordinatensystems, bezüglich dessen die Position und/oder Orientierung von Referenzobjekten des Körpers, wie insbesondere charakteristischen Referenzpunkten, Referenzachsen oder -geraden oder Referenzebenen, definiert ist, zumindest nahezu ausgerichtet ist. Dabei können die Referenzobjekte in dem Knochen-Koordinatensystem ausgewählt werden, so dass das Knochen- und das Referenz-Koordinatensystem ineinander überführbar oder transformierbar sind.

Vorzugsweise wird nach dem Ausrichten oder Vorjustieren des Basis-Koordinatensystems relativ zu dem Referenzkoordinatensystem die Ortungsreferenz von der Justierungseinrichtung entfernt, d.h. die Ortungsreferenz ist bevorzugt nur so lange an der Justierungseinrichtung angeordnet, bis der Ausrichtungs oder Vorjustierungsvorgang beendet ist.

Weiter wird nach der Ausrichtung oder Vorjustierung mittels der Recheneinheit die Position und/oder Orientierung der Ist-Schnittebene der Schneidführung relativ zu den der Recheneinheit bekannten oder von der Recheneinheit ermittelten Positionen und/oder Orientierungen der Referenzobjekte ermittelt. Die ermittelten Positionen oder Abstände der Ist-Schnittebene relativ zu oder von den Referenzobjekten werden vorzugsweise ausgegeben oder angezeigt, so dass beispielsweise ein Operateur basierend auf der ermittelten Position und/oder Orientierung der Ist-Schnittebene relativ zu den Referenzobjekten durch Veränderung der Justierungseinrichtung die Position und/oder Orientierung der Schnittebene bezüglich der Referenzobjekte verändert bis die Schneidführung oder Ist-Schnittebene vorbestimmte oder vorgegebene Abstände von den Referenzobjekten aufweist.

Bevorzugt wird mittels der Vorjustierungseinrichtung das Basis-Koordinatensystem so bezüglich des Referenz-Koordinatensystems ausgerichtet oder vorjustiert, dass eine erste Achse des Basis-Koordinatensystems parallel zu einer durch zwei Achsen des Referenz-Koordinatensystems aufgespannten Ebene verläuft und eine zweite Achse des Basis-Koordinatensystems parallel zu einer weiteren der Achsen oder der dritten Achse des Referenz-Koordinatensystems verläuft.

Nach dem Ausrichten oder Vorjustieren des Basis-Koordinatensystems bezüglich des Referenz-Koordinatensystems kann durch Veränderung eines Einstellmittels der Justierungseinrichtung die Schneidführung oder die Ist-Schnittebene relativ zu oder in dem Referenz-Koordinatensystem zumindest teilweise in nur einem Freiheitsgrad verändert werden. Das Verstellen eines ersten mechanischen Freiheitsgrades der Schneidführung führt zu einer Veränderung eines zu dem ersten mechanischen Freiheitsgrad der Schneidführung korrespondierenden ersten Freiheitsgrad der Ist-Schnittebene in dem Referenz-Koordinatensystem und zu einer Veränderung zumindest eines weiteren oder zweiten Freiheitsgrades der Ist-Schnittebene. Ist der erste Freiheitsgrad vorzugsweise wie gewünscht eingestellt, bewirkt eine Veränderung jedes weiteren mechanischen Freiheitsgrades der Schneidführung nur eine Veränderung des korrespondierenden oder entsprechenden Freiheitsgrades der Ist-Schnittebene. Nach dem Ausrichten oder Vorjustieren des Basis-Koordinatensystems bezüglich des Referenz-Koordinatensystems und nach dem Einstellen des ersten Freiheitsgrades kann die Schneidführung oder die Ist-Schnittebene bezüglich des Referenz-Koordinatensystems oder in dem Referenz-Koordinatensystem in voneinander entkoppelten Freiheitsgraden bewegt werden, so dass eine einfache und schnelle Ausrichtung der Ist-Schnittebene relativ zu den Referenzobjekten ermöglicht wird.

Die Offenbarung bezieht sich des Weiteren auf ein Computerprogramm, welches, wenn es in einen Computer geladen ist oder auf einem Computer läuft, das Verfahren durchführt sowie auf ein Programmspeichermedium oder Computerprogrammprodukt zur Ausführung des Programms.

Die Erfindung soll weiter anhand bevorzugter Ausführungsformen beschrieben werden.
Es zeigen:
- Figur 1: eine Justierungseinrichtung 20a eines herkömmlichen Schneidblocks;
- Figur 2: eine Justierungs- und Vorjustierungseinrichtung 20, 30 gemäß einer Ausführungsform der vorliegenden Erfindung;
- Figur 3: die Vorjustierungseinrichtung 30 aus Figur 2 während des Vorjustierungs-Vorganges
- Figuren 4a-4c: einen Ausrichtungsvorgang eines Basis- Koordinatensystems B auf ein Referenz-Koordinatensystem R während des Vorjustierungsvorganges;
- Figuren 5a - 5b: einen Ausrichtungsvorgang zweier Achsen des Basis-Koordinatensystems B mittels zweier Gelenke G1, G2 der Vorjustierungs-einrichtung;
- Figuren 6a-6e: einen Navigationsvorgang der Schnittebene 10 des Schneidblocks bezüglich Referenzen eines Körpers; und
- Figur 7: eine Positionsbestimmung der Schnittebene 10 bezüglich der Referenzen.

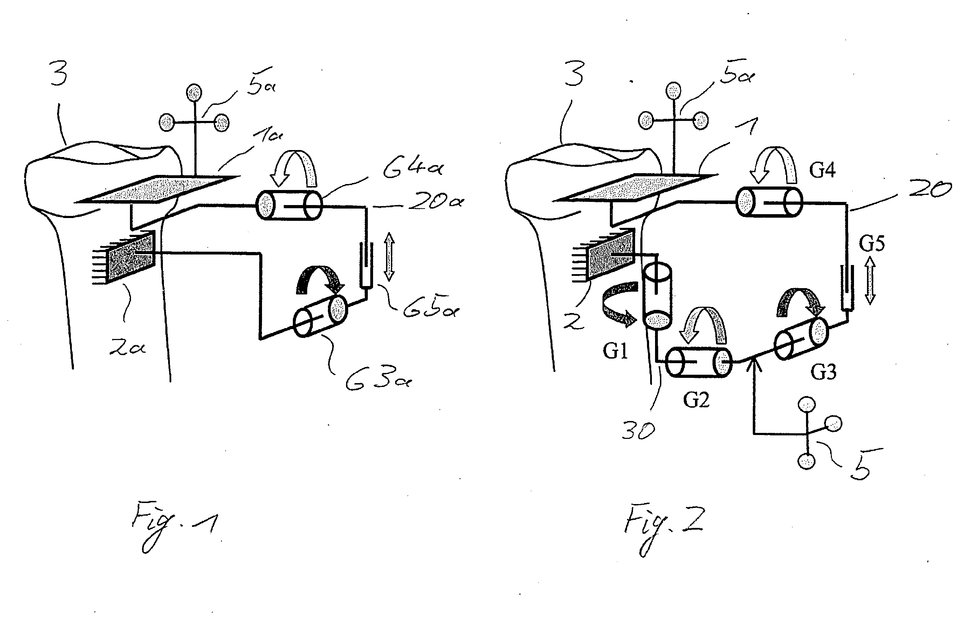

Figur 1 zeigt eine Justierungseinrichtung 20a eines herkömmlichen Schneidblocks des Stands der Technik. Über eine Befestigung 2a ist der Schneidblock an einem Knochen 3 angeordnet. Die Justierungseinrichtung 20a ist zwischen einer Schneidführung 1a und der Befestigung 2a angeordnet und umfasst drei Gelenke G3a, G4a, G5a. Die Gelenke G3a, G4a, G5a sind mit Einstellmitteln des Schneidblocks, wie Handrad-Einstellschrauben verbunden, so dass durch Einstellen eines Einstellmittels eine Bewegung des zugehörigen oder zugeordneten Gelenks der Gelenke G3a, G4a, G5a hervorgerufen wird. Durch eine Bewegung der Einstellmittel und damit der Gelenke G3a, G4a, G5a kann die Schneidführung 1a entsprechend bewegt werden, so dass sich die Position und/oder Orientierung oder Lage der Schneidführung 1a verändert. Über die zwei Gelenke G3a, G4a können rotatorische Bewegungen ausgeführt werden, so dass die Schneidführung 1a entsprechend der Veränderung der Einstellmittel und damit der Gelenke G3a, G4a gedreht wird. Über das Gelenk G5a können translatorische Bewegungen von dem Einstellmittel auf die Schneidführung 1a übertragen werden, so dass die Schneidführung 1a Translationsbewegungen ausführen kann. Die Position der Schneidführung 1a kann z.B. mit einem medizinischen Navigationssystem mit Hilfe eines an der Schneidführung angeordneten IR-Stahlung emittierenden oder reflektierenden Referenzsterns 5a oder einer andersartigen Ortungsreferenz oder eines andersartigen Registrierungselements überwacht werden. Durch Veränderung der Einstellmittel kann die Schneidführung 1a so ausgerichtet werden, dass die tatsächliche in dieser Position der Schneidführung 1a resultierende Ist-Schnittebene durch den Knochen 3 einer geplanten oder beispielsweise von einem Operateur vorgegebenen Soll- oder Plan-Schnittebene entspricht. Die Ist- und Soll-Schnittebene ist im Navigationssystem bezüglich eines mit der Justierungseinrichtung 20a verbundenen Basis-Koordinatensystems definiert, so dass die Lage der Ist- und der Soll-Schnittebene bezüglich des Basis-Koordinatensystems bekannt ist und Veränderungen der Lage der Ist- und der Soll-Schnittebene ermittelt und überwacht werden können. Versucht man jedoch keine solche Navigation der Ist-Schnittebene bezüglich eines vorbestimmten Plans, wie die Ausrichtung auf die oder bezüglich der Soll-Schnittebene durchzuführen, sondern stattdessen die Ist-Schnittebene bezüglich in einem Referenz-Koordinatensystem definierten Referenzen oder Referenzobjekten in dem Knochen 3 auszurichten oder zu navigieren, so wird der Navigationsvorgang mit dem herkömmlichen Schneidblock erheblich erschwert und verlangsamt. Wird nämlich die Position und/oder Orientierung der Schneidführung 1a des Schneidblocks in einem Freiheitsgrad bewegt oder verändert, so verändert sich die Position der Ist-Schnittebene bezüglich des an oder in dem Knochen 3 virtuell platzierten Referenz-Koordinatensystems, bezüglich dessen die Referenzen definiert sind, in zumindest einem weiteren Freiheitsgrad als dem gewünschten Freiheitsgrad, da die Drehachsen der Mechanik der Justierungseinrichtung 20a und des Referenz-Koordinatensystems verschieden sind und nicht aufeinander ausgerichtet sind. Damit müssen unbeabsichtigte Veränderungen oder Verstellungen der Ist-Schnittebene bezüglich des Referenz-Koordinatensystems korrigiert werden, wobei jede der Korrekturbewegungen eines Einstellmittels nicht nur eine Bewegung der Ist-Schnittebene in dem gewünschten sondern auch in zumindest einem weiteren nicht gewünschten Freiheitsgrad hervorruft. Somit ist eine Navigation bezüglich Referenzen mit einem herkömmlichen Schneidblock nur mit mehrfachen Iterationen oder in praktischen, notwendigerweise bezüglich ihrer Geschwindigkeit optimierten Anwendungen gar nicht möglich.

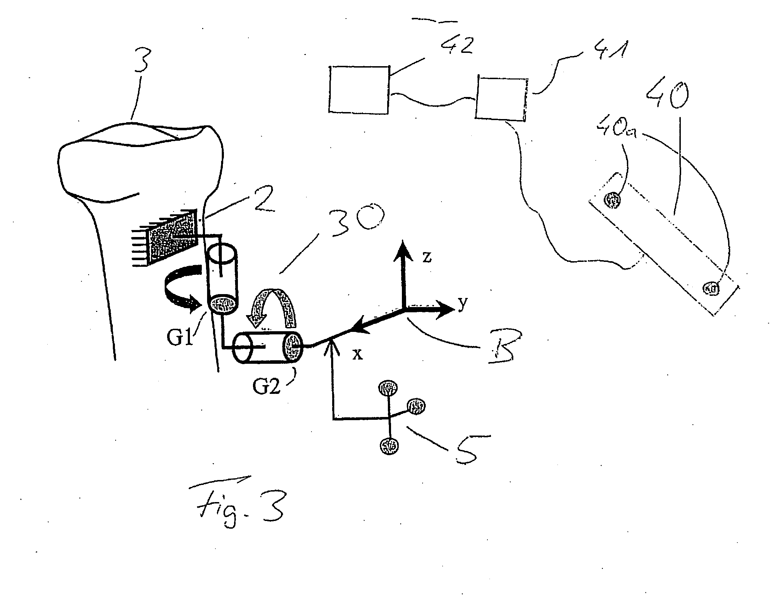

Figur 2 zeigt eine Ausführungsform einer Vorjustierungseinrichtung 30 eines Schneidblocks. Wie bezüglich Figur 1 erläutert, ist der Schneidblock mittels einer Befestigung 2 an einem Knochen 3 angebracht. Zusätzlich zu der drei Gelenke G3, G4, G5 umfassenden Justierungseinrichtung 20 ist zwischen der Justierungseinrichtung 20 und der Befestigung 2 eine Vorjustierungseinrichtung 30 an dem Schneidblock angeordnet. Die Vorjustierungseinrichtung 30 umfasst z.B. ein Kugelgelenk, z.B. realisiert durch zwei Scharnier-Gelenke G1, G2, über welche Rotatationsbewegungen übertragen werden können. Mit jedem der Gelenke G1, G2 ist jeweils ein Einstellmittel, insbesondere eine Handrad-Einstellschraube, der Vorjustierungseinrichtung 30 verbunden, welche ein Benutzer verstellen und drehen kann, so dass die Veränderung eines Einstellmittels eine Drehung oder Bewegung um das damit verbundene Gelenk der Gelenke G1, G2 bewirkt. Die Position und/oder Orientierung der Justierungseinrichtung 20 und der Vorjustierungseinrichtung 30 kann mit einem medizinischen Navigationssystem 40 ermittelt werden, so dass dem Navigationssystem 40 die Lage der Justierungseinrichtung 20 und der Vorjustierungseinrichtung 30 bekannt ist, indem das Navigationssystem 40 mittels IR-Kameras 40a einen an der Justierungseinrichtung 20 und der Vorjustierungseinrichtung 30 angeordneten Referenzstern 5 oder eine andersartige Ortungsreferenz oder ein andersartiges Registrierungselement erfasst, siehe Figur 3. Die Recheneinheit 41 des Navigationssystems 40 kann virtuell ein Basis-Koordinatensystem B definieren oder wählen, welches mit der Vorjustierungseinrichtung 30 so verbunden ist, dass eine Drehung eines der Gelenke G1, G2 eine Drehung des Basis-Koordinatensystems B bewirkt. Der Referenzstern 5 ist nur so lange an der Vorjustierungseinrichtung 30 angeordnet bis das Basis-Koordinatensystem B weit genug oder wie gewünscht gedreht wurde oder ausgerichtet wurde. Anschließend wird der Referenzstern 5 von der Vorjustierungseinrichtung 30 entfernt.

In Figur 2 ist eine drehbare Ortungsreferenz 5a an der Schneidführung 1a abgebildet.

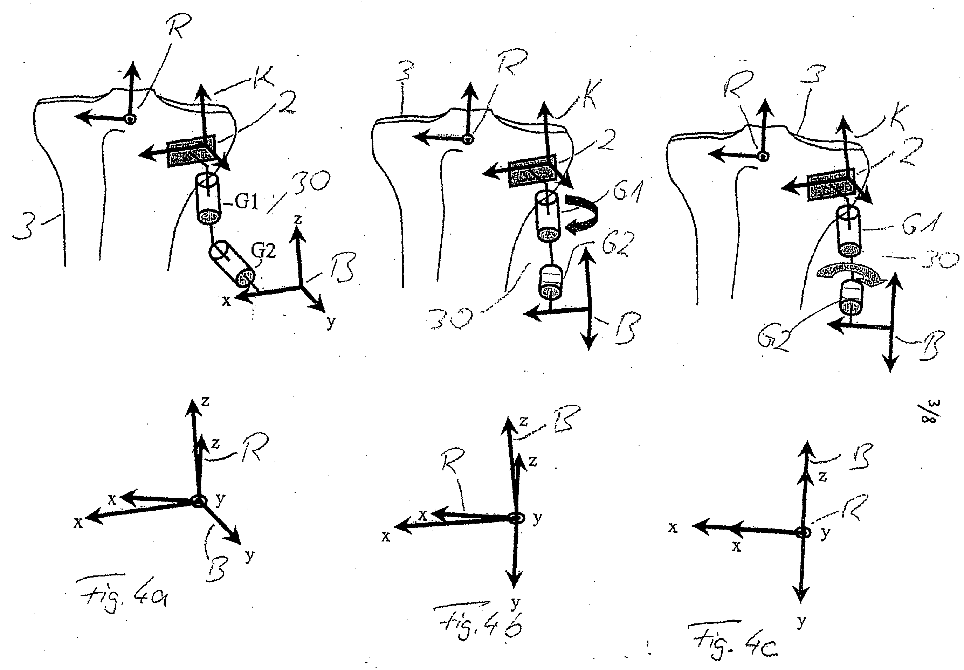

Mittels der Einstellmittel der Vorjustierungseinrichtung 30 wird das Basis-Koordinatensystem B so lange um die entsprechenden Gelenke G1, G2 bewegt oder gedreht, bis das Basis-Koordinatensystem B auf oder relativ zu einem Referenz-Koordinatensystem R ausgerichtet ist, welches von dem Navigationssystem 40 an oder in dem Knochen 3 definiert ist und bezüglich dessen Referenzen oder Referenzobjekte des Knochens 3 oder eines Körpers eines Patienten definiert sind. Zunächst sind, wie in Figur 4a zu erkennen, das Basis- B und das Referenz-Koordinatensystem R nicht aufeinander ausgerichtet. Durch Drehung des mit dem Gelenk G1 verbundenen Einstellmittels wird das Basis-Koordiantensystem B um das Gelenk G1 so lange gedreht bis man mit Hilfe des Navigationssystems 40 erkennen kann, dass die y-Achse des Basis-Koordinatensystems B in der y-z-Ebene des Referenz-Koordinatensystems R liegt, siehe Figur 4b. Alternativ kann auch die x-Achse des Basis-Koordinatensystems B in die x-y-Ebene des Referenzkoordinatensystems R gedreht werden oder es kann die z-Achse des Basis-Koordinatensystems B in die x-z-Ebene des Referenz-Koordinatensystems R gedreht werden. In einem weiteren Schritt wird das mit dem Gelenk G2 verbundene Einstellmittel der Vorjustierungseinrichtung 30 so lange gedreht bis mit Hilfe des Navigationssystems 40 erkannt werden kann, dass die x-Achse des Basis-Koordinatensystems B auf der x-Achse des Referenz-Koordinatensystems R liegt, siehe Figur 4c. Wurden zuvor die anderen Ausrichtungsmöglichkeiten gewählt kann alternativ in diesem Schritt die z-Achse des Basis-Koordinatensystems B auf die z-Achse des Referenz-Koordinatensystems R gedreht werden oder die y-Achse des Basis-Koordinatensystems B auf die y-Achse des Referenz-Koordinatensystems R gedreht werden. Die Ausrichtung ist dann, wie in Figur 4c zu erkennen, abgeschlossen, wenn die x-Achse des Basis-Koordinatensystems B auf der x-Achse des Referenz-Koordinatensystems R liegt und die y-Achse des Basis-Koordinatensystems B in der y-z-Ebene des Referenz-Koordinatensystems R liegt. Damit ist die Ausrichtung in zwei Schritten, durch Drehung um die Gelenke G1 und G2 abgeschlossen. In den Figuren 4a bis 4c liegt das Knochen-Koordinatensystem K an der Befestigung des Schneidblocks. Das Knochen-Koordinatensystem K kann aber auch ein beliebige andere Position an dem Knochen haben. Insbesondere steht die Lage des Knochen-Koordinatensystems K fest oder wird die Lage des Knochen-Koordinatensystems K definiert, bevor der Schneidblock an dem Knochen angebracht oder angeordnet wird.

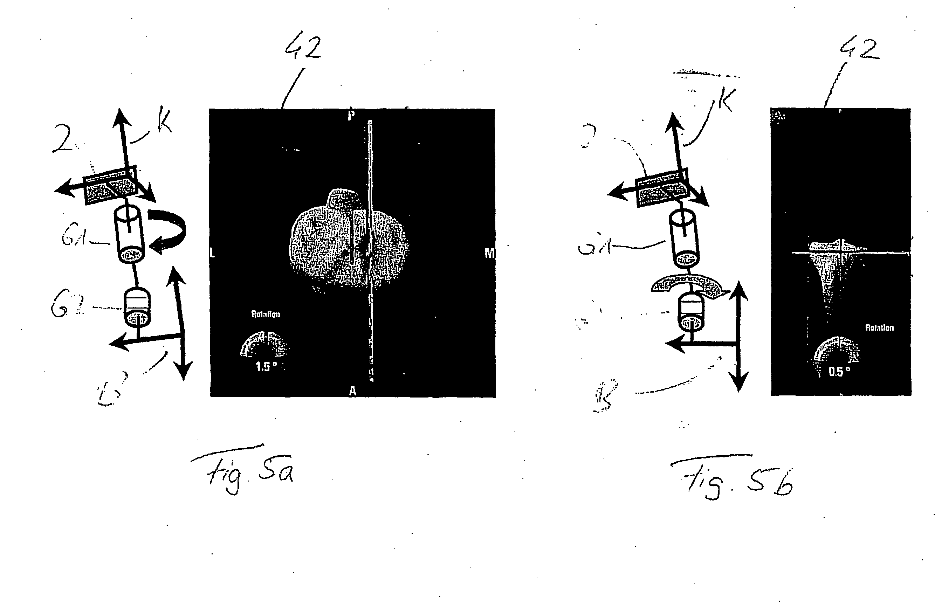

In den Figuren 5a und 5b ist zu erkennen, wie die Ausrichtung zweier Achsen des Basis-Koordinatensystems B mittels zweier Gelenke G1, G2 der Vorjustierungseinrichtung an dem Navigationssystem, z.B. mittels einer auf einem Bildschirm 42 angezeigten oder dargestellten Darstellung des Knochens und der zwei Achsen des Basis-Koordinatensystems B, dargestellt oder visualisiert werden kann. Weiter ist in den Figuren 5a und 5b ein Knochen-Koordinatensystem K zu erkennen, bezüglich dessen die Lage der Schneidführung 1a definiert ist.

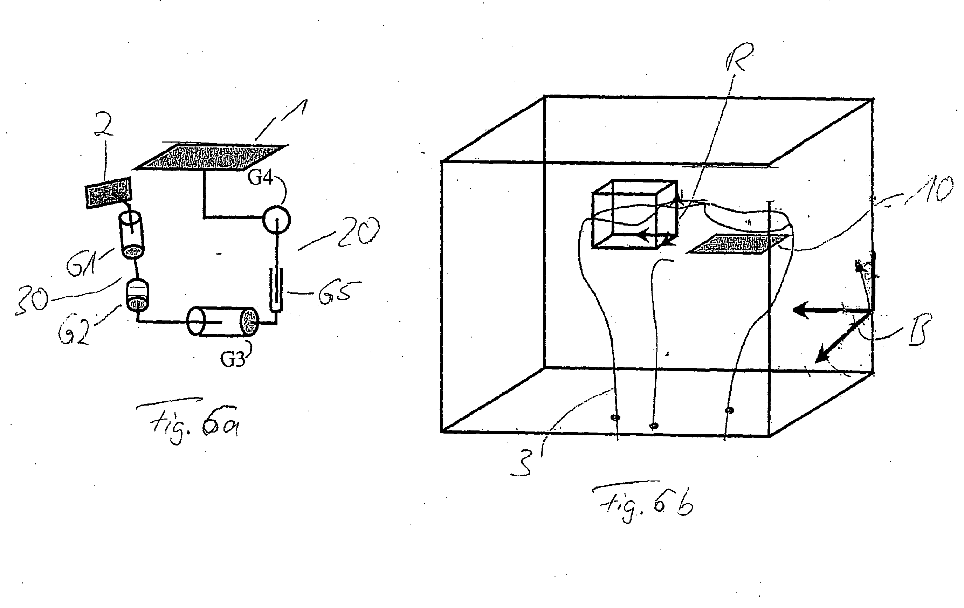

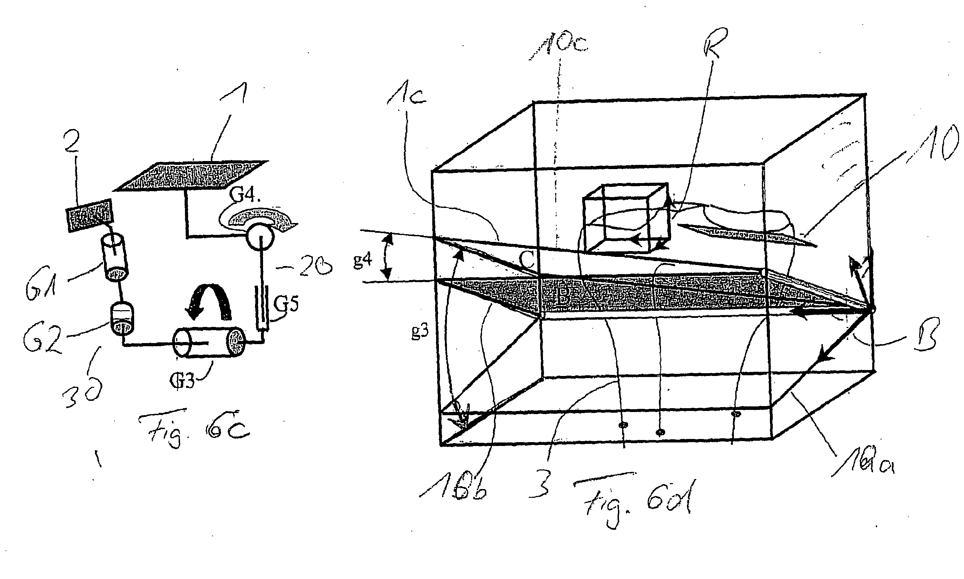

Ist nun, wie in Figur 6b zu erkennen, das Basis-Koordinatensystem B auf das Referenz-Koordinatensystem R ausgerichtet, kann die Schneidführung 1 und damit die Ist-Schnittebene 10, welche eine beliebige Ausgangslage z.B. wie in Figur 6b oder wie in Figur 6d dargestellt haben kann, des Schneidblocks mittels der Justierungseinrichtung 20 durch Verstellen der mit den Gelenken G3, G4, G5 verbunden Einstellmittel, siehe Figur 6a, bezüglich der Referenzobjekte eingestellt werden. Die Lage der Ist-Schnittebene 10 kann mittels des Navigationssystems mit Hilfe der an der Schneidführung 1 angeordneten Ortungsreferenz, z.B. eines Referenzsterns, ermittelt werden.

In dem Navigationssystem 40 wird zunächst die Ausgangslage der Ist-Schnittebene 10, insbesondere relativ zu dem Knochen 3, ermittelt und z.B. auf einem Monitor 42 angezeigt. In Figur 6d befindet sich die Ist-Schnittebene 10 in der Ausgangslage 10a. Durch Verstellen des mit dem Gelenk G3 der Justierungseinrichtung 20 verbundenen Einstellmittels, wie in Figur 6c zu sehen, wird die Ist-Schnittebene 10 aus der Position 10a in die Position 10b gedreht, so dass zunächst die von einem Operateur gewünschte oder vorgegebene Flexion/Extension eingestellt wird. Durch Verstellen des mit dem Gelenk G4 verbundenen Einstellmittels wird die Ist-Schnittebene 10 aus der Position 10b in die Position 10c bewegt, bis der vorgegebene Varus/Valgus-Winkel erreicht ist. Durch Einstellen des mit dem Gelenk G5 verbundenen Einstellmittels wird schließlich die vorgegebene Resektion eingestellt. Bei allen Einstellungen wird der Operateur durch das Navigationssystem 40 unterstützt, indem beim Verstellen der Einstellmittel die Veränderung der Flexion/Extension, des Vaurus/Valgus-Winkels und der Resektion ermittelt, überwacht und ausgegeben wird, so dass der Operateur einfach und schnell die gewünschten Werte einstellen kann.

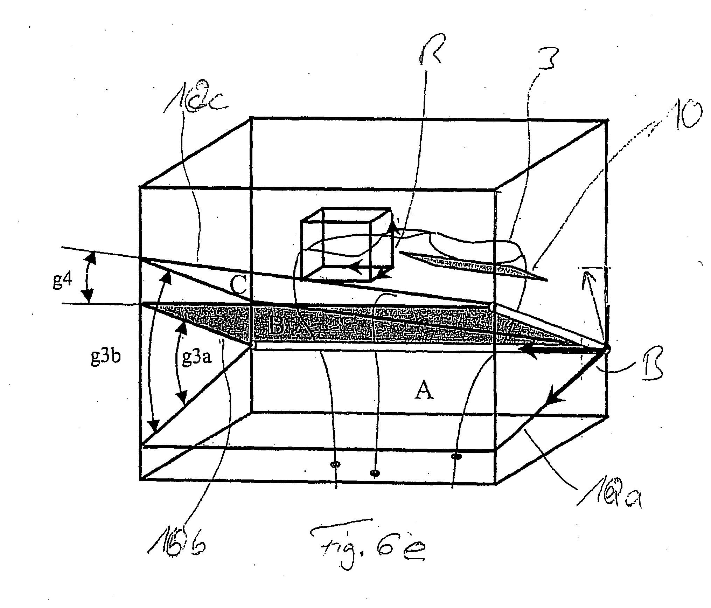

Wie zuvor beschrieben, wird zunächst der gewünschte Flexionswinkel durch Verstellen des mit dem Gelenk G3 verbundenen Einstellmittels eingestellt. Da das Basis-Koordinatensystem B bezüglich des Referenz-Koordinatensystems R zumindest nahezu ausgerichtet wurde, bleibt, wie in Figur 6e zu erkennen, bei der anschließenden Änderung des Varuswinkels mit dem mit dem Gelenk G4 verbundenen Einstellmittel der Flexionswinkel g3a vor der Einstellung des Varuswinkels gleich dem Flexionswinkel g3b nach der Varuswinkel-Einstellung. Somit können die Freiheitsgrade der Ist-Schnittebene 10 in dem Referenz-Koordinatensystem R entkoppelt voneinander geändert werden.

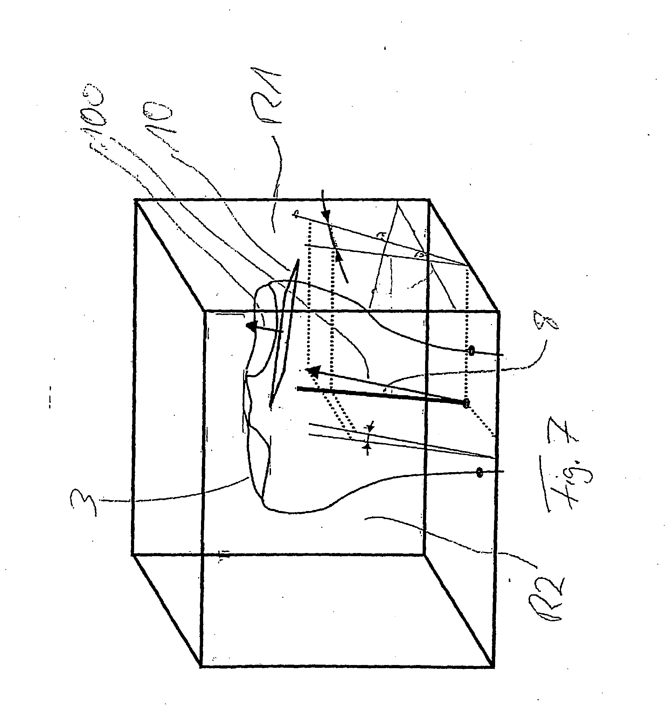

Zur Einstellung des Flexions-Extensionswinkels und des Varus-Valgus-Winkels werden, wie in Figur 7 erkennbar, die Winkel zwischen einer als Referenz dienenden Achse 8 und der Schnittnormale 100 der Ist-Schnittebene auf die Sagittalebene R1 und die Frontalebene R2, durch welche das Referenz-Koordinatensystem R aufgespannt wird, projiziert und nach Wunsch oder wie von einem Operateur angegeben, eingestellt. Alternativ können auch andere Ebenen als Projektionsebenen verwendet werden und das Referenz-Koordinatensystem R aufspannen.

## Patentansprüche

1. Schneidblock mit :
- einer Schneidführung (1), an welcher verstellbar, insbesondere drehbar, eine Ortungsreferenz (5a) angebracht ist, mittels welcher die Ist-Schnittebene (10) der Schneidführung (1) räumlich, insbesondere bezüglich eines an einem Knochen (3) angeordneten Knochen-Koordinatensystems (K), bestimmt werden kann,
- einer Befestigung (2), die an einem Knochen (3) eines Körpers eines Patienten befestigt werden kann,
- einer Justierungseinrichtung (20), wobei mittels der Justierungseinrichtung (20) die Ist-Schnittebene (10) der Schneidführung (1) gegenüber dem Knochen (3) mit zwei rotatorischen Freiheitsgraden und einem translatorischen Freiheitsgrad eingestellt werden kann, wobei die Lage der Justierungseinrichtung (20) bezüglich eines Basis-Koordinatensystems (B) definiert ist,
- wobei die Position und/oder Orientierung von Referenzobjekten (8) des Körpers, wie Referenzpunkten, Referenzachsen oder -geraden oder Referenzebenen, bezüglich eines Referenz-Koordinatensystems (R) definiert sind,
**gekennzeichnet durch**
- eine Vorjustierungseinrichtung (30), mittels welcher das Basis-Koordinatensystem (B) des Schneidblocks bezüglich des Referenz-Koordinatensystems (R) mit zwei rotatorischen Freiheitsgraden so ausgerichtet werden kann, dass
- eine erste Achse des Basis-Koordinatensystems (B) parallel zu einer **durch** zwei Achsen des Referenz-Koordinatensystems (R) aufgespannten Ebene verläuft und eine zweite Achse des Basis-Koordinatensystems (B) parallel zu der dritten Achse des Referenz-Koordinatensystems (R) verläuft.

2. Schneidblock nach Anspruch 1, wobei die Vorjustierungseinrichtung (30) mit einer Ortungsreferenz (5) verbunden ist.

3. Schneidblock nach einem der vorhergehenden Ansprüche, wobei die Vorjustierungseinrichtung (30) Vorjustierungs-Einstellmittel, insbesondere jeweils ein Einstellmittel für jeden Freiheitsgrad, speziell zwei Handrad-Schraubeneinstellungen oder arretierbare Schrauben, aufweist, mittels denen die Schneidführung (1) um zwei nichtparallele Achsen gedreht werden kann, so dass das Basis-Koordinatensystem (B) bezüglich des Referenz-Koordinatensystems (R) zumindest nahezu ausgerichtet werden kann.

4. Schneidblock nach einem der vorhergehenden Ansprüche, wobei die Vorjustierungseinrichtung (30) zwischen der Befestigung (2) des Schneidblocks und der Justierungseinrichtung (20) ausgebildet ist.

5. Schneidblock nach einem der vorhergehenden Ansprüche, wobei die Vorjustierungseinrichtung (30) Gelenke, insbesondere Kugelgelenke oder Scharniergelenke, umfasst, um welche das Basis-Koordinatensystem (B) gedreht werden kann.

## Claims

1. A cutting block, comprising:
- a cutting guide (1) to which a localisation reference (5a) is adjustably, in particular rotatably, attached, by means of which the actual incision plane (10) of the cutting guide (1) can be spatially determined, in particular with respect to a bone co-ordinate system (K) arranged on a bone (3);
- a fixation (2) which can be fixed to a bone (3) of a patient's body;
- an adjusting device (20), wherein by means of the adjusting device (20), the actual incision plane (10) of the cutting guide (1) relative to the bone (3) can be set with two rotational degrees of freedom and one translational degree of freedom, and wherein the location of the adjusting device (20) is defined with respect to a base co-ordinate system (B),
- wherein the position and/or orientation of reference objects (8) of the body, such as reference points, reference axes, reference straight lines or reference planes, is/are defined with respect to a reference co-ordinate system (R),
**characterised by**
- a pre-adjusting device (30), by means of which the base co-ordinate system (B) of the cutting block can be aligned with respect to the reference co-ordinate system (R) with two rotational degrees of freedom, such that
- a first axis of the base co-ordinate system (B) runs parallel to a plane spanned by two axes of the reference co-ordinate system (R), and a second axis of the base co-ordinate system (B) runs parallel to the third axis of the reference co-ordinate system (R).

2. The cutting block according to claim 1, wherein the pre-adjusting device (30) is connected to a localisation reference (5).

3. The cutting block according to any one of the preceding claims, wherein the pre-adjusting device (30) comprises pre-adjusting setting means, in particular one setting means for each degree of freedom respectively, specifically two hand wheel screw setting devices or locking screws by means of which the cutting guide (1) can be rotated about two non-parallel axes, such that the base co-ordinate system (B) can be at least almost aligned with respect to the reference co-ordinate system (R).

4. The cutting block according to any one of the preceding claims, wherein the pre-adjusting device (30) is formed between the fixation (2) of the cutting block and the adjusting device (20).

5. The cutting block according to any one of the preceding claims, wherein the pre-adjusting device (30) comprises joints, in particular ball joints or hinged joints, about which the base co-ordinate system (B) can be rotated.

## Revendications

1. Bloc de coupe comportant :
- un guide de coupe (1) sur lequel une référence de localisation (5a) est fixée de manière ajustable, en particulier de manière à pouvoir tourner, au moyen de laquelle le plan de coupe réel (10) du guide de coupe (1) peut être déterminé dans l'espace, en particulier par rapport à un système de coordonnées d'os (K) agencé sur un os (3),
- une fixation (2) qui peut être fixée sur un os (3) d'un corps de patient,
- un dispositif d'ajustement (20), le dispositif d'ajustement (20) permettant de régler le plan de coupe réel (10) du guide de coupe (1) par rapport à l'os (3) avec deux degrés de liberté de rotation et un degré de liberté de translation, la position du dispositif d'ajustement (20) étant définie par rapport à un système de coordonnées de base (B),
- dans lequel la position et/ou l'orientation d'objets de référence (8) du corps, tels que des points de référence, des axes ou des droites de référence ou des plans de référence, sont définies par rapport à un système de coordonnées de référence (R),
**caractérisé par**
- un dispositif de pré-ajustement (30) au moyen duquel le système de coordonnées de base (B) du bloc de coupe peut être orienté par rapport au système de coordonnées de référence (R) avec deux degrés de liberté de rotation de telle sorte que
- un premier axe du système de coordonnées de base (B) s'étend parallèlement à un plan défini par deux axes du système de coordonnées de référence (R) et un deuxième axe du système de coordonnées de base (B) s'étend parallèlement au troisième axe du système de coordonnées de référence (R).

2. Bloc de coupe selon la revendication 1, dans lequel le dispositif de pré-ajustement (30) est relié à une référence de localisation (5).

3. Bloc de coupe selon l'une quelconque des revendications précédentes, dans lequel le dispositif de pré-ajustement (30) comporte des moyens de réglage de pré-ajustement, en particulier un moyen de réglage pour chaque degré de liberté, notamment deux moyens de réglage par vis de volant de manoeuvre ou vis blocables, au moyen desquels le guide de coupe (1) peut être mis en rotation autour de deux axes non parallèles de telle sorte que le système de coordonnées de base (B) peut être sensiblement orienté au moins par rapport au système de coordonnées de référence (R).

4. Bloc de coupe selon l'une quelconque des revendications précédentes, dans lequel le dispositif de pré-ajustement (30) est formé entre la fixation (2) du bloc de coupe et le dispositif d'ajustement (20).

5. Bloc de coupe selon l'une quelconque des revendications précédentes, dans lequel le dispositif de pré-ajustement (30) comporte des articulations, en particulier des articulations sphériques ou des articulations à charnière, autour desquelles le système de coordonnées de base (B) peut tourner.
